# DEMANDE DE BREVET EUROPEEN

(11) **EP 2 690 170 A1**
(43) Date de publication de la demande: **29.01.2014**
(21) Numéro de dépôt: 13305836.2
(22) Date de dépôt: 20.06.2013
(51) Int. Cl.: C12N 1/20, A62D 3/02, C12R 1/01

(54) **Bactéries du genre Pseudonocardia capables de dégrader le méthyl tert-butyl éther (MTBE) ou l'éthyl tert-butyl éther (ETBE) en solution dans des effluents**

(30) Priorité: 25.07.2012 FR 1202105
(71) Demandeur: IFP Energies nouvelles, 92852 Rueil-Malmaison Cedex (FR)
(72) Inventeur: Le Digabel, Yoann, 92800 Puteaux (FR); Fayolle-Guichard, Francoise, 75005 Paris (FR)

(57) **Abrégé**

La présente invention concerne des bactéries du genre *Pseudonocardia,* et en particulier la souche déposée le 12 juillet 2012 à l'Institut Pasteur (CNCM, 25 rue du Docteur Roux, F-75724 PARIS Cedex 15, France), sous le n° CNCM I-4656, capables de dégrader le MTBE et/ou l'ETBE.

L'invention porte également sur un procédé de traitement d'un effluent comprenant du MTBE et/ou de l'ETBE et éventuellement du TBA mettant en oeuvre de telles bactéries.

## Description

La présente invention concerne des microorganismes du genre *Pseudonocardia* capables de dégrader des additifs des essences et en particulier l'éthyl *tert*-butyl éther (ETBE) ou le méthyl *tert*-butyl éther (MTBE) ou le tert-butyl alcool (TBA) en solution dans l'eau. L'invention a également pour objet une souche du genre *Pseudonocardia* déposée, le 12 juillet 2012 à l'Institut Pasteur (CNCM, 25 rue du Docteur Roux, F-75724 PARIS Cedex 15, France) sous le n° CNCM I-4656. Les microorganismes selon l'invention trouvent leur application dans l'industrie du traitement des eaux.

La présente invention a également pour objet un procédé de traitement d'effluents aqueux contenant des composés du type éthyl *tert*-butyl éther (ETBE) et/ou méthyl *tert-*butyl éther (MTBE) ou éventuellement du tert-butyl alcool (TBA) en mettant en oeuvre de tels microorganismes.

### Etat de la technique

Il est connu que des additifs sont ajoutés aux essences pour améliorer les performances des moteurs, c'est le cas des additifs oxygénés, ou éthers-carburants : le méthyl tert-butyl éther (désigné ci-après sous le terme de MTBE) est un des éthers qui peut être utilisé comme additif oxygéné dans les essences sans plomb dans le but d'augmenter leur indice d'octane ainsi que de l'éthyl tert-butyl éther (désigné ci-après sous le terme d'ETBE) qui est préférentiellement utilisé depuis plusieurs années en France et en Europe du fait de sa qualification en tant que biocarburant. Ces composés peuvent être ajoutés aux essences à raison de 22% (v/v). Le transport d'hydrocarbures, par voie terrestre ou maritime, présente de nombreux risques d'accidents. Le transport par pipe-lines qui est généralement considéré comme plus sûr que par camion, train ou tanker peut néanmoins induire des pollutions. Il a été estimé en 2010 que les pipelines représentent la source la plus fréquente (27 %) des déversements. Les pollutions terrestres par les hydrocarbures sont également dues à des accidents de camions ou de trains pendant le transport, des accidents durant le remplissage des cuves de stations-service, des fuites sur des cuves de stockage dans des stations-service ou sur des sites industriels. En plus de ces sources majeures de pollution par hydrocarbures, il existe une pollution chronique qui se produit durant le remplissage des réservoirs des véhicules dans les stations-service ou à des fuites sur les réservoirs des véhicules. Dans ces deux derniers cas, cette décharge vers les eaux terrestres de très faible quantité de manière chronique est aussi importante. Parmi les composés des essences, tous n'ont pas la même toxicité et/ou biodégradabilité et ceci va déterminer leur devenir dans l'environnement. Le benzène, par exemple, qui est un des composés monoaromatiques des essences, est un composé très toxique mais facilement dégradé en aérobiose. Parmi les composés natifs des essences qui sont récalcitrants à la biodégradation, on peut citer le 2,2,4-triméthylpentane (désigné sous le terme d'isooctane) ou le cyclohexane, dont les niveaux de toxicité sont un peu moindres.

La littérature relative à la biodégradation des composés des essences ou des alcanes par les microorganismes est importante. De nombreux microorganismes ayant des capacités de dégradation de ces composés ont été isolés. Par contre, un nombre plus restreint de microorganismes avec des capacités de dégradation du MTBE ou de l'ETBE, dont la biodégradation est plus lente que celle des composés dit "facilement biodégradables", ont été isolés. En raison de l'utilisation croissante d'additifs tels que le MTBE ou l'ETBE dans les formulations d'essences ou de gazole, il est donc nécessaire de connaître le devenir de ces composés en cas de déversement accidentel conduisant à une pollution des sols et des eaux souterraines ou de surface. Cette nécessité est d'autant plus grande dans le cas du MTBE et de l'ETBE car ces composés sont très solubles dans l'eau (40 et 10 g.L⁻¹, respectivement) et qu'ils sont considérés comme potentiellement toxiques (en particulier le MTBE) et que, hormis toute considération de toxicité, leur présence dans l'eau à de très faibles concentrations rend l'eau impropre à la consommation du fait du goût qu'ils lui confèrent. Par ailleurs, en raison de leur faible biodégradabilité, la contamination par un de ces composés peut conduire à leur persistance comme cela a récemment été démontré par exemple sur un site de station-service contaminé par de l'ETBE, où des concentrations jusqu'à 200 mg.L⁻¹ d'ETBE ont été mesurées même après plusieurs années de traitement du site par les moyens classiques (sparging,...), confirmant ainsi la faible efficacité des moyens physico-chimiques classiques pour la réhabilitation de tels sites *[*Journal of Hazardous Materials, 201-202 (2012) p. 36-243*].*

Un but de l'invention est de proposer de nouveaux microorganismes capables de biodégrader le MTBE ou l'ETBE qui peuvent atteindre les nappes aquifères dans les cas de pollution.

### Résumé de l'invention

La Demanderesse a découvert de façon surprenante que les microorganismes du genre *Pseudonocardia* avaient des capacités de biodégradation importantes du MTBE et/ou de l'ETBE et/ou le TBA, en particulier la souche *Pseudonocardia* déposée le 12 juillet 2012 à l'Institut Pasteur (CNCM, 25 rue du Docteur Roux, F-75724 PARIS Cedex 15) sous la n° CNCM I-4656.

### Description détaillée de l'invention

La Demanderesse a observé de façon surprenante que lorsque l'on fournit à une bactérie du genre *Pseudonocardia,* et en particulier à la souche *Pseudonocardia* déposée le 12 juillet 2012 sous le n° CNCM I-4656, de l'ETBE ou du MTBE, celle-ci s'est avérée capable de dégrader ces deux composés. Le *tert*-butyl alcool (TBA) qui est un intermédiaire majeur de la dégradation du MTBE et de l'ETBE est produit transitoirement au cours de la biodégradation puis est ensuite totalement consommé par cette bactérie prouvant ainsi que les bactéries selon l'invention possède la totalité des enzymes de dégradation permettant d'aller jusqu'à la minéralisation et la production de biomasse.

Les bactéries selon l'invention ont été isolées à partir de microcosmes provenant de différents environnements qui ont été obtenus par enrichissement sur un milieu minimum contenant le MTBE ou l'ETBE comme seule source de carbone. Ce protocole a été réalisé selon les techniques d'enrichissement en microorganismes spécifiques connues de l'homme de l'art.

Les souches bactériennes résultantes ont été isolées après ces étapes d'enrichissement spécifiques sur des boîtes de pétri contenant du milieu riche classiquement utilisé par l'homme de l'art (milieu Tripticase/Soja ou TS) mais après dilution de ce milieu au 1/10 par rapport à la concentration habituellement utilisée. Ces bactéries ont ensuite été identifiées d'après leur séquence d'ADNr 16S et par comparaison avec les banques de données des ADN bactériens puis elles ont été testées pour leurs capacités de dégradation de l'ETBE et le MTBE.

La présente invention concerne également un procédé de traitement d'effluents contenant du MTBE et/ou de l'ETBE et éventuellement du TBA dans lequel on met en contact en conditions aérobies l'effluent en présence au moins d'une souche bactérienne du genre *Pseudonocardia,* et en particulier la souche *Pseudonocardia* déposée le 12 juillet 2012 sous le n° CNCM I-4656 à l'Institut Pasteur (25 rue du Docteur Roux, F-75724 PARIS Cedex 15, France).

La mise en oeuvre de ces bactéries pour le traitement en continu d'effluents pollués par du MTBE ou de l'ETBE peut être réalisée, par exemple dans un biofiltre où les bactéries sont fixées sur un support minéral ou organique ou bien elles peuvent être ajoutées comme inoculum à des boues de station d'épuration, ou dans tout autre système adapté au traitement des eaux et des sols (biobarrière).

Selon un mode de réalisation avantageux, le procédé de traitement peut mettre en oeuvre les bactéries selon l'invention en association avec les bactéries décrites dans la demande de brevet FR 2 944 006, lorsque l'effluent contient un cocktail de composés hydrocarbonés tels que l'octane, le benzène, l'éthylbenzène, le toluène, le *m*-xylène, le p-xylène, le cyclohexanol, le cyclohexane, l'isooctane.

### Brève description des figures

Ces aspects ainsi que d'autres aspects de l'invention seront clarifiés dans la description détaillée de modes de réalisation particuliers de l'invention, référence étant faite aux dessins des figures, dans lesquelles :

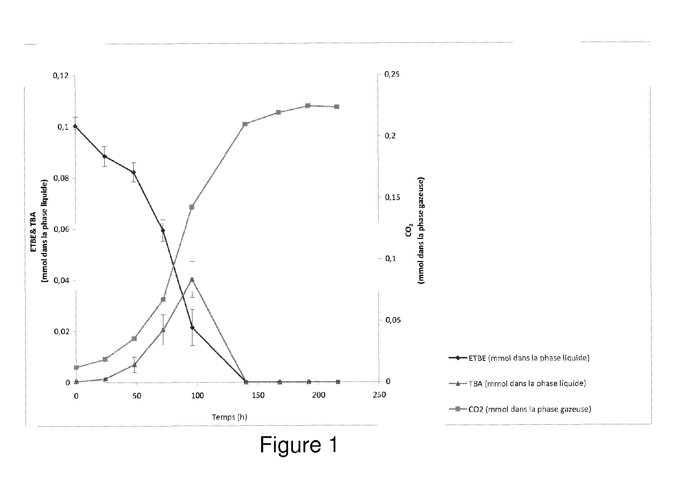

- la figure 1 montre l'évolution de la concentration en ETBE, en TBA dans le milieu culture et du CO₂ formé en fonction du temps de contact avec la bactérie du genre *Pseudonocardia* déposée le 12 juillet 2012 sous le n° CNCM I-4656 à l'Institut Pasteur (25 rue du Docteur Roux, F-75724 PARIS Cedex 15, France).

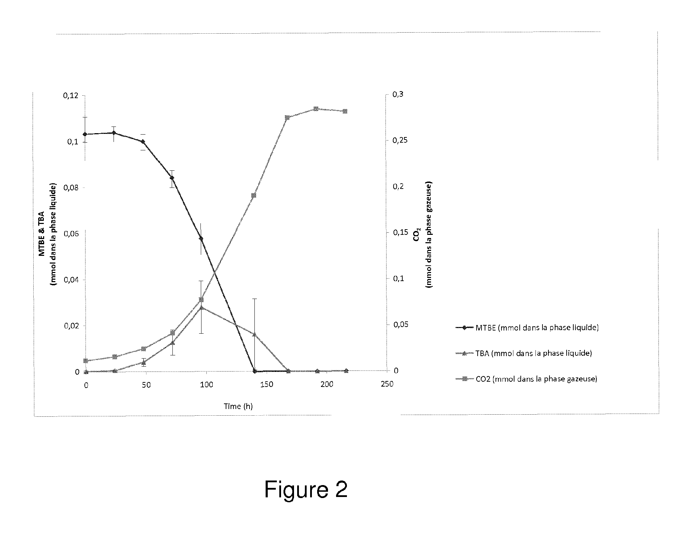

- la figure 2 montre l'évolution de la concentration en MTBE, en TBA dans le milieu culture et du CO₂ formé en fonction du temps de contact avec la bactérie du genre *Pseudonocardia* déposée le 12 juillet 2012 sous le n° CNCM I-4656 à l'Institut Pasteur (25 rue du Docteur Roux, F-75724 PARIS Cedex 15, France).

### Exemples

### Exemple 1: Croissance de la bactérie du genre Pseudonocardia déposée le 12 juillet 2012 sous le n° CNCM I-4656 à l'Institut Pasteur (25 rue du Docteur Roux, F-75724 PARIS Cedex 15, France) sur un milieu minéral en présence d'ETBE comme seule source de carbone

On effectue une préculture de la bactérie du genre *Pseudonocardia* n° CNCM I-4656 : on ensemence la souche *Pseudonocardia* n° CNCM I-4656 sur milieu minéral salin MM supplémenté en ETBE à environ 200 mg.L⁻¹ comme source de carbone et d'énergie.

Le milieu MM a la composition suivante :

| | |
|---|---|
| KH₂PO₄ | 1,4 g |
| K₂HPO₄ | 1,7 g |
| NaNO₃ | 1,5 g |
| MgSO₄, 7H₂O | 0,5 g |
| CaCl₂, 2H₂O | 0,04 g |
| FeCl₃, 6H₂O | 0,012 g |
| Solution concentrée de vitamines | 1 mL |
| Solution concentrée d'oligoéléments | 1 mL |
| H₂O | 1 L |

La solution concentrée de vitamines a la composition suivante pour 1 litre d'eau distillée :

| | |
|---|---|
| Biotine | 200 mg |
| Riboflavine | 50 mg |
| Acide nicotinamique | 50 mg |
| Panthoténate | 50 mg |
| Acide p-aminobenzoïque | 50 mg |
| Acide folique | 20 mg |
| Thiamine | 15 mg |
| Cyanocobalamine | 1,5 mg |

La solution concentrée d'oligoéléments a la composition suivante pour 1 litre d'eau distillée :

| | |
|---|---|
| CuSO₄, 5 H₂O | 0,1 g |
| MnSO₄, 2H₂O | 1 g |
| ZnSO₄, 7 H₂O | 1 g |
| AlCl₃, 6H₂O | 0,4 g |
| NiCl₂,6 H₂O | 0,25 g |
| H₃BO₃ | 0,1 g |
| CoCl₂, 6 H₂O | 1 g |
| Na₂MoO₄, 2H₂O | 1 g |
| Na₂WO₄, 2H₂O | 1 g |

Après croissance, cette préculture est utilisée pour ensemencer 100 mL du milieu minéral salin (taux d'ensemencement : 10% v/v) auquel on ajoute de l'ETBE à une concentration finale d'environ 300 mg.L⁻¹ dans une fiole d'Erlenmeyer d'une contenance de 500 mL fermée avec un bouchon téflonné afin d'éviter toute perte d'ETBE au cours de la croissance. Un prélèvement est effectué au temps t=0 pour un dosage de l'ETBE au départ par analyse en chromatographie en phase gazeuse avec un détecteur à ionisation de flamme (CPG/FID). La fiole est ensuite incubée à 30°C dans un agitateur rotatif. Un prélèvement pour le dosage du substrat et de ses éventuels produits de dégradation est effectué à intervalle régulier. La production de CO₂ produit dans la phase gazeuse est également mesurée par prélèvement de ladite phase gazeuse à travers le septum avec une seringue à gaz étanche par analyse du CO₂ en chromatographie en phase gazeuse avec un détecteur de type catharomètre (CPG/TCD).

Le résultat de cette expérimentation est présenté dans la figure 1. Comme on le voit sur cette figure, l'ETBE est dégradé en partie en TBA. Ce composé est ensuite lui-même re-consommé et utilisé comme substrat de croissance.

On peut appliquer le protocole décrit ci-dessus à différentes bactéries pour évaluer leur capacité a dégrader l'ETBE et donc permet de sélectionner les bactéries capables de dégrader l'ETBE.

### Exemple 2: Croissance de la bactérie du genre Pseudonocardia déposée le 12 juillet 2012 sous le n° CNCM I-4656 à l'Institut Pasteur (25 rue du Docteur Roux, F-75724 PARIS Cedex 15, France) sur un milieu minéral en présence de MTBE comme seule source de carbone

On effectue une préculture de la bactérie du genre *Pseudonocardia* n° CNCM I-4656 : on ensemence la souche *Pseudonocardia* n° CNCM I-4656 sur milieu minéral salin MM supplémenté en MTBE à environ 200 mg.L⁻¹ comme source de carbone et d'énergie.. Le milieu MM a la composition décrite dans l'exemple 1.

Après croissance, cette préculture est utilisée pour ensemencer 100 mL de milieu MM (taux d'ensemencement : 10% v/v) auquel on ajoute du MTBE à une concentration finale d'environ 300 mg.L⁻¹ dans une fiole d'Erlenmeyer d'une contenance de 500 mL fermée avec un bouchon téflonné afin d'éviter toute perte de MTBE au cours de la croissance. Un prélèvement est effectué au temps t=0 pour un dosage du MTBE au départ par analyse en chromatographie en phase gazeuse avec un détecteur à ionisation de flamme (CPG/FID). La fiole est ensuite incubée à 30°C dans un agitateur rotatif. Un prélèvement pour le dosage du substrat et de ses éventuels produits de dégradation est effectué à intervalle régulier. La production de CO₂ produit dans la phase gazeuse est également mesurée par prélèvement de ladite phase gazeuse à travers le septum avec une seringue à gaz étanche par analyse du CO₂ en chromatographie en phase gazeuse avec un détecteur de type catharomètre (CPG/TCD).

Le résultat de cette expérimentation est présenté dans la figure 2. Comme on le voit sur cette figure 2, le MTBE est dégradé en partie en TBA. Ce composé est ensuite lui-même re-consommé et utilisé comme substrat de croissance.

On peut appliquer le protocole décrit ci-dessus à différentes bactéries pour évaluer leur capacité a dégrader le MTBE et donc permet de sélectionner les bactéries capables de dégrader le MTBE.

## Revendications

1. Bactérie du genre *Pseudonocardia* capable de dégrader le MTBE et/ou l'ETBE.

2. Bactérie du genre *Pseudonocardia* déposée le 12 juillet 2012 sous le n° ° CNCM I-4656 à l'Institut Pasteur (25 rue du Docteur Roux, F-75724 PARIS Cedex 15, France)

3. Procédé de traitement d'un effluent comprenant du MTBE et/ou du ETBE et éventuellement du TBA comme substrat de croissance dans lequel on met en contact, dans des conditions aérobies, ledit effluent avec au moins une bactérie selon la revendication 2.

4. Procédé selon la revendication 3 dans lequel la bactérie est fixée dans un biofiltre.

5. Procédé selon la revendication 4 dans lequel la bactérie est fixée sur un support minéral ou organique.

6. Procédé selon la revendication 3 dans lequel on met en contact l'effluent avec une boue d'épuration dans laquelle a été inoculée la bactérie selon la revendication 2.
